(19) **Europäisches Patentamt / European Patent Office / Office européen des brevets**

(11) **EP 3 956 464 B1**

(12) **EUROPEAN PATENT SPECIFICATION**

(45) Date of publication and mention of the grant of the patent:
**06.12.2023 Bulletin 2023/49**

(21) Application number: **20723791.8**

(22) Date of filing: **15.04.2020**

(51) International Patent Classification (IPC):
**C12Q 1/68** (2018.01)

(52) Cooperative Patent Classification (CPC):
**C12Q 1/6886; C12Q 2600/154**

(86) International application number:
**PCT/EP2020/060517**

(87) International publication number:
**WO 2020/212378 (22.10.2020 Gazette 2020/43)**

(54) **IN VITRO METHOD FOR THE DIAGNOSIS OR PROGNOSIS OF COLORECTAL CANCER OR A PRE-CANCEROUS STAGE THEREOF**

IN-VITRO-VERFAHREN ZUR DIAGNOSE ODER PROGNOSE VON KOLOREKTALEM KREBS ODER EINEM PRÄKANZERÖSEN STADIUM DAVON

PROCÉDÉ IN VITRO POUR LE DIAGNOSTIC OU LE PRONOSTIC DU CANCER COLORECTAL ET/OU D'UN STADE PRÉCANCÉREUX ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.04.2019 EP 19382290**

(43) Date of publication of application:
**23.02.2022 Bulletin 2022/08**

(73) Proprietors:
• **Servizo Galego De Saúde**
  **15703 Santiago de Compostela (ES)**
• **Fundación Instituto de Investigación Sanitaria de Santiago de Compostela**
  **15706 Santiago de Compostela (ES)**

(72) Inventors:
• **DÍAZ LAGARES, Ángel**
  **15706 Santiago de Compostela (ES)**
• **LÓPEZ LÓPEZ, Rafael**
  **15706 Santiago de Compostela (ES)**
• **CRUJEIRAS MARTÍNEZ, Ana Belén**
  **15706 Santiago de Compostela (ES)**
• **RODRÍGUEZ CASANOVA, Aitor**
  **15706 Santiago de Compostela (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(56) References cited:
**CN-A- 106 755 464      CN-A- 109 504 778**
**US-A1- 2018 112 272**

• N LI ET AL: "Phosphodiesterase 10A: a novel target for selective inhibition of colon tumor cell growth and [beta]-catenin-dependent TCF transcriptional activity", ONCOGENE, vol. 34, no. 12, 7 April 2014 (2014-04-07) , pages 1499-1509, XP055630811, London ISSN: 0950-9232, DOI: 10.1038/onc.2014.94
• HUI CHEN ET AL: "Long noncoding RNA LNC473 inhibits the ubiquitination of survivin via association with USP9X and enhances cell proliferation and invasion in hepatocellular carcinoma cells", BIOCHEMICAL AND BIOPHYSICAL RESEARCH COMMUNICATIONS, vol. 499, no. 3, 1 May 2018 (2018-05-01), pages 702-710, XP55713704, AMSTERDAM, NL ISSN: 0006-291X, DOI: 10.1016/j.bbrc.2018.03.215

- **WANG YANAN ET AL: "LncRNA AB073614 regulates proliferation and metastasis of colorectal cancer cells via the PI3K/AKT signaling pathway", BIOMEDICINE AND PHARMACOTHERAPY, ELSEVIER, FR, vol. 93, 20 July 2017 (2017-07-20), pages 1230-1237, XP085147893, ISSN: 0753-3322, DOI: 10.1016/J.BIOPHA.2017.07.024**
- **GUO TINGTING ET AL: "Multidimensional communication of microRNAs and long non-coding RNAs in lung cancer", JOURNAL OF CANCER RESEARCH AND CLINICAL ONCOLOGY, SPRINGER INTERNATIONAL, BERLIN, DE, vol. 145, no. 1, 11 November 2018 (2018-11-11), pages 31-48, XP036669792, ISSN: 0171-5216, DOI: 10.1007/S00432-018-2767-5 [retrieved on 2018-11-11]**

Remarks:
    The complete document including Reference Table(s) and the Sequence Listing(s) can be downloaded from the EPO website

**Description**

**FIELD OF THE INVENTION**

The present invention refers to the medical field. Particularly, the present invention refers to an *in vitro* method for the diagnosis and/or prognosis of colorectal cancer (CRC) and/or a pre-cancerous stage thereof. The method of the invention comprises the determination of the methylation status of the gene *LINC00473,* preferably in a liquid biopsy obtained from the patient, wherein a higher level of methylation of the gene *LINC00473* with respect to an established reference control level, is indicative of the presence of colorectal cancer, a pre-cancerous stage thereof and/or of poor prognosis of the disease.

**STATE OF THE ART**

CRC, also known as colon cancer, rectal cancer, or bowel cancer, is the development of cancer in the colon or rectum (parts of the large intestine). The vast majority of colorectal cancers are adenocarcinomas. This is because the colon has numerous glands within the tissue. When these glands undergo a number of changes at the genetic level, they proceed in a predictable manner as they move from benign to an invasive, malignant colon cancer. The adenomas of the colon, particularly advanced colorectal adenoma (AA), are a benign version of the malignant adenocarcinomas but still with malignant potential if not removed (they are usually removed because of their tendency to become malignant and to lead to colon cancer).

Screening is an effective way for preventing and decreasing deaths from colorectal cancer and is recommended starting from the age of 50 to 75. The best known and most frequently used screening test for colorectal cancer is called Fecal Immunochemical Test (FIT). FIT detects blood in the stool samples which can be a sign of pre-cancer or cancer. If abnormal results are obtained, usually a colonoscopy is recommended which allows the physician to look at the inside of the colon and rectum to make a diagnosis. During colonoscopy, small polyps may be removed if found. If a large polyp or tumor is found, a biopsy may be performed to check if it is cancerous. The gastroenterologist uses a colonoscopy to find and remove these adenomas and polyps to prevent them from continuing to acquire genetic changes that will lead to an invasive adenocarcinoma.

Although, as explained above, FIT is nowadays used for screening colorectal cancer, it is important to note that FIT offers a low sensitivity for AA which means that most of said kind of patients can be wrongly classified as not having the disease. Consequently, FIT is not able to identify adenomas due to its low sensitivity. Moreover, since FIT uses stool samples, it offers a low compliance. On the other hand, colonoscopy is an invasive technique wherein the most severe complication generally is the gastrointestinal perforation. Moreover, colonoscopy is nowadays a procedure involving anesthesia, and the laxatives which are usually administered during the bowel preparation for colonoscopy are associated with several digestive problems. It is important to note that the methods used today for screening general population at risk of suffering for CRC or AA are associated with a high rate of false positives. Consequently, a high amount of unnecessary follow-up colonoscopies are nowadays performed.

The present invention offers a clear solution to the problems cited above because it is focused on an *in vitro* method for identifying or screening human subjects at risk of suffering from colorectal cancer or colorectal adenomas (particularly advanced colorectal adenomas). Since the method of the invention is preferably based on a liquid biopsy obtained from the patient (for example plasma, blood or serum), it is expected to improve compliance to colorectal cancer screening. Moreover, the method of the invention offers high sensitivity and specificity, which means that it is a strong and cost-effective method for the detection of both colorectal cancer and colorectal adenomas. Li et al (Oncogene, 2014) discloses that the expression of PDE10a (in D3 it is referred to as PDE10 but in the first line of the abstract it is identified as PDE10a) is increased in colon cancer and if the expression is increased in gain of function experiments, the normal and precancerous tissue turns more carcinogeneous. Chen et al (Biochemical and Biophysical Research communications,2018) discloses Lnc00473 expression as being associated with hepatocellular cancer proliferation and invasion.

**DESCRIPTION OF THE INVENTION**

**Brief description of the invention**

The present invention refers to an *in vitro* method (hereinafter "method of the invention") for the diagnosis or prognosis of colorectal cancer and/or a pre-cancerous stage thereof. It is preferably implemented in a liquid biopsy obtained from the patient, which is a minimally-invasive biological sample. The method of the invention offers high sensitivity and specificity, which means that it is a strong and cost-effective method for the detection of both colorectal cancer and/or a pre-cancerous stage thereof.

Since the method of the invention has higher sensitivity and specificity as compared to the method used today (FIT) for screening general population at risk of suffering from CRC or AA, it is associated with a lower percentage of false positives. Consequently, the method described in the present invention clearly helps in reducing the number of follow-up colonoscopies, thus improving the way that the patients are nowadays screened or diagnosed. Once the method of the invention is performed, if it is determined that the patients might be suffering from colorectal cancer and/or precancerous stage, the result is confirmed by colonoscopy. However, if it is not determined that the patient might be suffering from colorectal cancer and/or precancerous stage, there is no need to perform a colonoscopy and routine testing with the method of the invention is recommended.

Particularly, the first embodiment of the present invention refers to an *in vitro* method for the diagnosis of colorectal cancer and/or a pre-cancerous stage thereof, which comprises determining the methylation status of at least the gene *LINC00473* in a liquid biopsy obtained from the patient, wherein a higher level of methylation of the gene *LINC00473,* as compared with a reference level of methylation of the gene *LINC00473* measured in healthy subjects, is an indication that the subject is suffering from colorectal cancer and/or a pre-cancerous stage thereof.

The second embodiment of the invention refers to an *in vitro* method for the prognosis of colorectal cancer and/or a pre-cancerous stage thereof, which comprises determining the methylation status of at least the gene *LINC00473* in a liquid biopsy obtained from the patient, wherein a higher level of methylation of the gene *LINC00473,* as compared with a reference level of methylation of the gene *LINC00473* measured in the patient, is an indication that the patient has a poor prognosis.

The third embodiment of the present invention refers to the *in vitro* use of at least the methylation status of the gene *LINC00473* for the diagnosis and/or prognosis of colorectal cancer and/or a pre-cancerous stage thereof.

The fourth embodiment of the present invention refers to the *in vitro* use of a kit comprising reagents for the determination of at least the methylation status of the gene *LINC00473* for the diagnosis and/or prognosis of colorectal cancer and/or a pre-cancerous stage thereof.

In a preferred embodiment, the liquid biopsy is a sample of plasma, blood or serum. Plasma is particularly preferred.

In a preferred embodiment, the pre-cancerous stage is colorectal adenoma, preferably AA.

In a preferred embodiment, the methylation status of the gene *LINC00473* is determined in at least a CpG of the promoter region.

In a preferred embodiment, the methylation status of the gene *LINC00473* is determined in at least a CpG of the promoter region which is 3000 bp around the transcription start site (TSS). This means 1500 bp upstream or 1500 bp downstream of the TSS.

In a preferred embodiment, the methylation status of the gene *LINC00473* is determined in at least a CpG of the promoter region located between the chromosomal positions Chr6: 166402081 and Chr6: 166402638.

In a preferred embodiment, the methylation status of the gene *LINC00473* is determined in at least a CpG of the promoter region located at a chromosomal position selected from the group comprising: Chr6: 166402638, and/or Chr6: 166402474, and/or Chr6: 166402463, and/or Chr6: 166402457, and/or Chr6: 166402416, and/or Chr6: 166402379, and/or Chr6: 166402375, and/or Chr6: 166402364, and/or Chr6: 166402081, preferably a CpG selected from the group comprising: cg06545143, and/or cg08886973 and/or cg21306006.

In a preferred embodiment, the diagnosis of the colorectal cancer and/or a pre-cancerous stage thereof is confirmed by an image technique, preferably colonoscopy.

According to the method of the invention, after measuring the methylation status of the gene *LINC00473,* a score value is obtained and this score value is compared with a threshold value which defines the diagnostic rule. If this score value is higher than the threshold, then the corresponding sample is classified as a positive sample, which is an indication that the patient might be suffering from colorectal cancer and/or pre-cancerous stage thereof. The threshold value has been defined in order to optimize sensitivity and specificity values. Consequently, in a preferred embodiment, the method of the invention comprises: a) Measuring methylation status of the gene *LINC00473,* in a liquid biopsy obtained from the subject, b) processing the methylation values in order to obtain a risk score and c) wherein if a deviation or variation of the risk score value obtained is identified, as compared with a reference value, this is indicative that the subject is suffering from colorectal cancer and/or a pre-cancerous stage thereof.

The last embodiment of the present invention refers to a method for the diagnosis and treatment colorectal cancer or a pre-cancerous stage thereof, which comprises: a) obtaining a liquid biopsy from a human subject; b) detecting the methylation status of the gene *LINC00473,* and c) diagnosing the patient with colorectal cancer or a pre-cancerous stage thereof when a higher level of methylation of the gene *LINC00473* is identified, as compared with a reference level of methylation of the gene *LINC00473* measured in healthy subjects, and performing a colonoscopy to the patient. The colonoscopy may comprise the removal of the colorectal cancer or the polyps.

For the purpose of the present invention the following terms are defined:

- *LINC00473* (Long Intergenic Non-Protein Coding RNA 473) is an RNA Gene and is affiliated with the non-coding RNA class. It can be identified in public data bases as follows: HGNC: 21160. Entrez Gene: 90632. Ensambl ID:

ENSG00000223414. UniProtKB: A8K010.

- The term "colorectal cancer" is a medical condition characterized by cancer of cells of the intestinal tract below the small intestine (i.e., the large intestine (colon), including the cecum, ascending colon, transverse colon, descending colon, sigmoid colon, and rectum).
- The expression "colorectal adenoma" refers to adenomas of the colon, also called adenomatous polyps, which is a benign and pre-cancerous stage of the colorectal cancer but still with high risk of progression to colorectal cancer.
- The expression "advanced colorectal adenoma" refers to adenomas having a size of at least 10 mm or histologically having high grade dysplasia or a villous component higher than 20%; or serrated lesions having a size of at least 10 mm or dysplasia. For a more detailed explanation of the concept "advanced colorectal adenoma" please refer to the following scientific articles which pertain to the common general knowledge in the field of the invention: [Rex DK, Boland CR, Dominitz JA, Giardiello FM, Johnson DA, Kaltenbach T, Levin TR, Lieberman D, Robertson DJ. Colorectal Cancer Screening: Recommendations for Physicians and Patients From the U.S. Multi-Society Task Force on Colorectal Cancer. Gastroenterology. 2017 Jul;153(1): 307-323. doi: 10.10531j.gastro.2017.05.013. Epub 2017 Jun 9. PMID: 28600072][East JE, Atkin WS, Bateman AC, Clark SK, Dolwani S, Ket SN, Leedham SJ, Phull PS, Rutter MD, Shepherd NA, Tomlinson I, Rees CJ. British Society of Gastroenterology position statement on serrated polyps in the colon and rectum. Gut. 2017 Jul;66(7):1181-1196. doi: 10.1136/gutjnl-2017-314005. Epub 2017 Apr 27. Review. PMID: 28450390].
- The expression "liquid biopsy" refers to any sample of biologic fluids that may contain tumor-derived material. Particularly, a liquid biopsy is any sample of biologic fluids (for example: plasma, serum, blood, saliva, cerebrospinal fluid or urine) that may contain tumor-derived material, such as circulating tumor DNA.
- The expression "minimally-invasive biological sample" refers to any sample which is taken from the body of the patient without the need of using harmful instruments, other than fine needles used for taking the blood from the patient, and consequently without being harmfully for the patient. Specifically, minimally-invasive biological sample refers in the present invention to: blood, serum, or plasma samples.
- As used herein, the term "methylation" will be understood to mean the presence of a methyl group added by the action of a DNA methyl transferase enzyme to a cytosine base or bases in a region of nucleic acid e.g. DNA.
- Accordingly, the term, "methylation status" as used herein refers to the level of methylation measured in the patient which is being analyzed. The "methylation status" may be a higher/lower level of methylation as compared with the reference or control level.
- As used herein, a "CpG dinucleotide", "CpG methylation site" or equivalent, shall be taken to denote a cytosine linked to a guanine by a phosphodiester bond. CpG dinucleotides are targets for methylation of the cytosine residue and may reside within coding or non-coding nucleic acids. Non-coding nucleic acids are understood in the art to include introns, 5'- untranslated regions, 3' untranslated regions, promoter regions of a genomic gene, or intergenic regions.
- The expression "target CpG" refers to the specific CpG which is found to be differentially methylated in the present invention.
- The expression "consecutive CpG" refers to CpGs which may be differentially methylated, and they can be found up to 1500 bp upstream or downstream of the transcription start site (TSS) (3000 bp around the TSS).
- As used in the present invention "determining the methylation status of the gene" means that the method of the invention is performed by measuring the methylation status of any region of the gene, also comprising any regulatory sequence which is capable of increasing or decreasing the expression of said genes. Particularly, said regulatory sequence includes the promoter which is a region of DNA that initiates transcription of the gene. Promoters are located near the transcription start sites of genes, on the same strand, upstream or downstream on the DNA (towards the 5' region of the sense strand).
- As used herein, a "control, reference or baseline level of methylation" shall be understood to mean a level of methylation detected in a corresponding nucleic acid from a healthy patient. Thus, the patient is likely to suffer from CRC or AA, with a given sensitivity and specificity, if a "higher level of methylation" is measured in this patient as compared with a "control, reference or baseline level of methylation" measured in a healthy patient. A "reference value" can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Preferably, the person skilled in the art may compare the biomarker levels (or scores) obtained according to the method of the invention with a defined threshold value. Typically, the optimal sensitivity and specificity (and so the threshold value) can be determined using a Receiver Operating Characteristic (ROC) curve based on experimental data. For example, after determining the methylation level of the biomarkers in a group of reference, one can use algorithmic analysis for the statistic treatment of the measured methylation level of biomarkers in biological samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is good. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0, preferably GraphPad Prism 6.

- The expression "higher level of methylation" refers to a statistically significant increase in the relative amount of methylation of a nucleic acid e.g., DNA, as compared with the amount of methylation measured in a subject/patient used as control/reference. Thus, in the present disclosure, the "higher level of methylation" is determined with reference to a baseline level represented by the methylation status of a given genomic region in a sample obtained from the subject or patient. For example, "higher level of methylation" may be at least 2% greater than the baseline level of methylation, for example at least 5% greater than the baseline level of methylation, or at least 10% greater than the baseline level of methylation, or at least 15% greater than the baseline level of methylation, or at least 20% greater than the baseline level of methylation, or at least 25% greater than the baseline level of methylation, or at least 30% greater than the baseline level of methylation, or at least 40% greater than the baseline level of methylation, or at least 50% greater than the baseline level of methylation, or at least 60% greater than the baseline level of methylation, or at least 70% greater than the baseline level of methylation, or at least 80% greater than the baseline level of methylation, or at least 90% greater than the baseline level of methylation.

- By "comprising" it is meant including, but not limited to, whatever follows the word "comprising". Thus, use of the term "comprising" indicates that the listed elements are required or mandatory, but that other elements are optional and may or may not be present.

- By "consisting of" it is meant "including, and limited to", whatever follows the phrase "consisting of". Thus, the phrase "consisting of" indicates that the listed elements are required or mandatory, and that no other elements may be present.

## Brief description of the figures

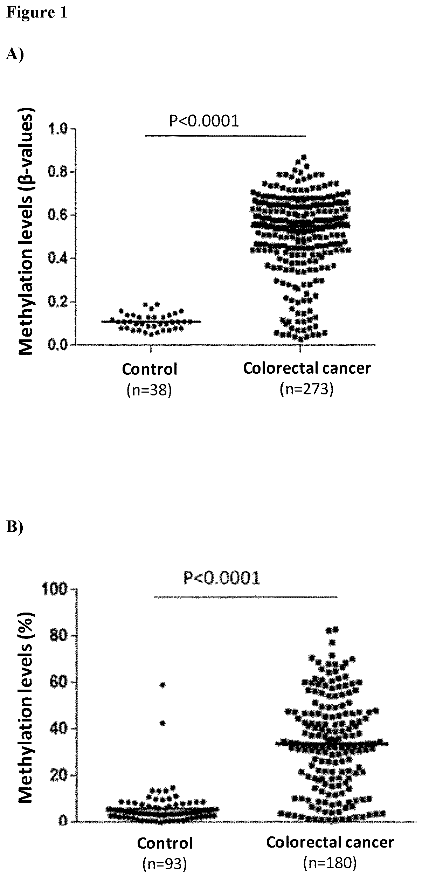

**Figure 1. DNA methylation levels of *LINC00473* promoter in colorectal tissue samples.** In cohort 1 (**A**) and 2 (**B**), colorectal cancer patients showed significantly higher methylation levels of *LINC00473* than non-tumor controls. The methylation levels of the *LINC00473* promoter were analyzed (**A**) by Infinium HumanMethylation450K BeadChip (450K array), and (**B**) by pyrosequencing. DNA methylation data are expressed as β-values in **A**, and as % of methylation in **B**. The horizontal lines represent the median of the methylation levels. P, indicates the p-value analyzed by Mann-Whitney U test. Tumor, represents colorectal cancer.

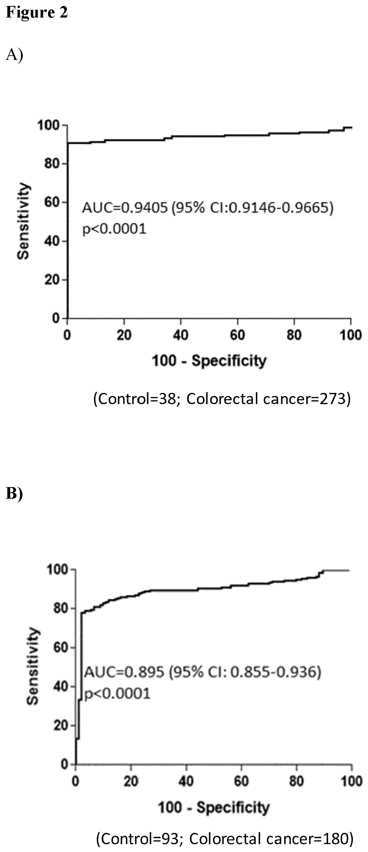

**Figure 2. Receiver Operating Characteristics (ROC) curve for the methylation of *LINC00473* promoter in colorectal tissue samples.** The area under the ROC curve (AUC) in the Cohort 1 (**A**) and Cohort 2 (**B**) show the high diagnostic accuracy of the methylation of *LINC00473* promoter to discriminate between colorectal cancer patients and non-tumor controls. P, indicates the p-value of the ROC curve. CI, is the confidence interval.

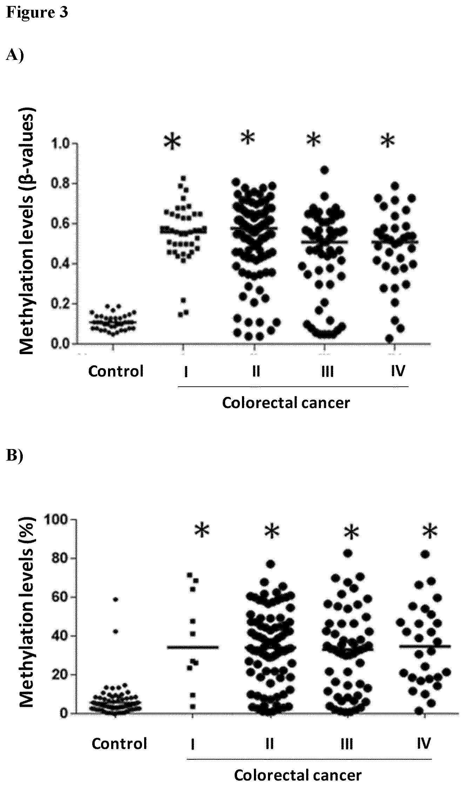

**Figure 3. DNA methylation levels of *LINC00473* promoter in colorectal tissue samples from The Cancer Genome Atlas (TCGA) according to their clinical stage.** All the stages of colorectal cancer patients showed significantly higher methylation levels of *LINC00473* in (**A**) Cohort 1 (I: 0.55 ± 0.023; II: 0.53 ± 0.018; III: 0.45 ± 0.028; IV: 0.47 ± 0.031) and (**B**) Cohort 2 (I: 38.54% ± 7,67%; II: 34.31% ± 2.11%; III: 32.14% ± 2.84%; IV: 35.31% ± 3.95%) than their respective non-tumor controls (Cohort 1: 0.11 ± 0.006; Cohort 2: 5.84% ± 0.78%). The methylation levels of *LINC00473* were analyzed (**A**) by Infinium HumanMethylation450K BeadChip (450K array) and expressed as β-values, and (**B**) by pyrosequencing and expressed as % of methylation. Asterisks (*), indicate P<0.0001 respect to controls. The p-value was analyzed by Mann-Whitney U test. Tumor, represents colorectal cancer.

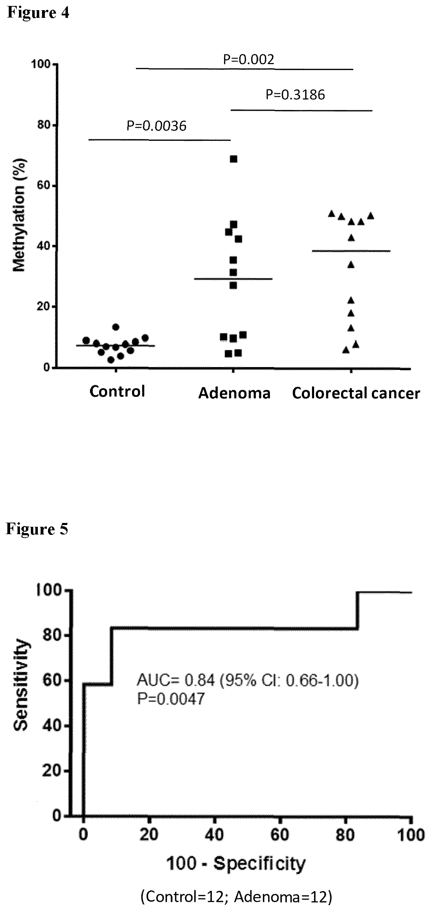

**Figure 4. DNA methylation levels of *LINC00473* promoter in adenomas.** Adenomas and colorectal cancer patients showed significantly higher methylation levels of *LINC00473* than non-tumor controls. The methylation levels of the *LINC00473* promoter were analyzed by pyrosequencing and expressed as % of methylation. The horizontal lines represent the median of the methylation levels. P, indicates the p-value analyzed by Mann-Whitney U test.

**Figure 5. Receiver Operating Characteristics (ROC) curve for the methylation of *LINC00473* promoter in adenomas.** The area under the ROC curve (AUC) in the Cohort 3 shows the high diagnostic accuracy of the methylation of *LINC00473* promoter to discriminate between adenomas and non-tumor controls. P, indicates the p-value of the ROC curve. CI, is the confidence interval.

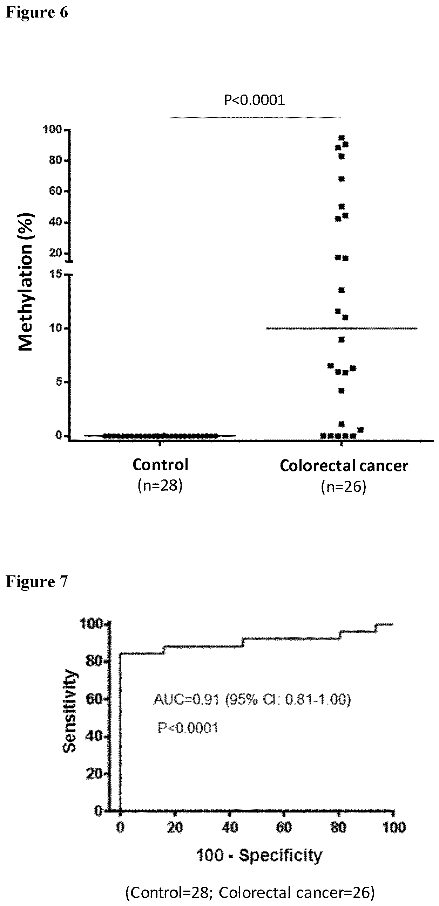

**Figure 6. DNA methylation levels of *LINC00473* promoter in circulating plasma DNA of colorectal cancer patients and healthy controls.** Colorectal cancer patients showed significantly higher methylation levels of *LINC00473* than healthy controls. The methylation levels of *LINC00473* were analyzed in triplicates by real-time PCR. The horizontal lines represent the median of the methylation levels. P, indicates the p-value analyzed by Mann-Whitney U test.

**Figure 7. Receiver Operating Characteristics (ROC) curve for the methylation of *LINC00473* promoter in circulating plasma DNA of colorectal cancer patients respect to normal controls.** The area under the ROC curve (AUC) shows the high diagnostic accuracy of the methylation of *LINC00473* promoter to discriminate between colorectal cancer patients and healthy individuals. P, indicates the p-value of the ROC curve.

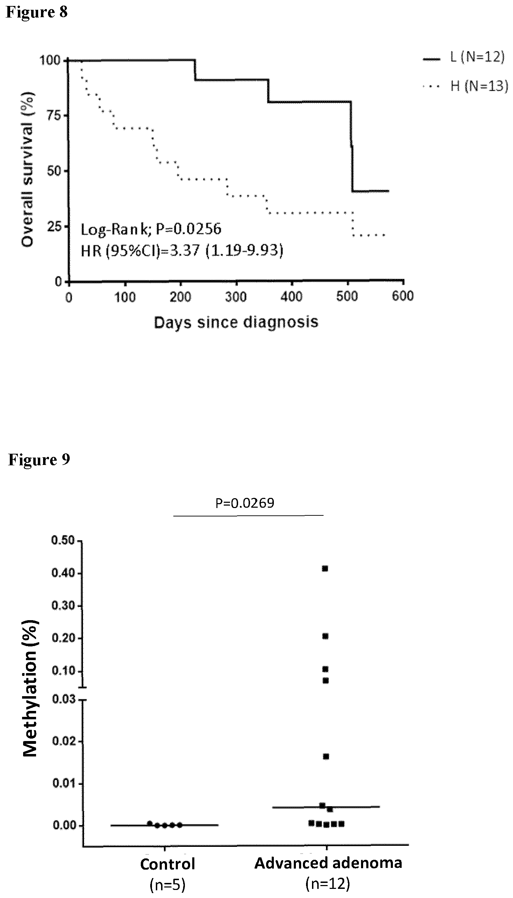

**Figure 8. Kaplan-Meier survival analysis for the methylation of *LINC00473* promoter in colorectal cancer patients.** Colorectal cancer patients were classified according to a higher or lower level of *LINC00473* methylation respect to the median methylation level of the group (10% of methylation). Kaplan-survival analyses showed that high methylation levels of *LINC00473* were significantly associated with shorter overall survival (OS) (P=0.0256). P, refers to the p-value of the Log-Rank test. H (High) indicates a methylation level >10%; and L (Low), a methylation level <10%.

**Figure 9. DNA methylation levels of *LINC00473* promoter in circulating plasma DNA of advanced colorectal adenoma patients and healthy controls.** Advanced colorectal adenoma patients showed significantly higher methylation levels of *LINC00473* than healthy controls. The methylation levels of *LINC00473* were analyzed in triplicates by real-time PCR. The horizontal lines represent the median of the methylation levels. P, indicates the p-value analyzed by Mann-Whitney U test.

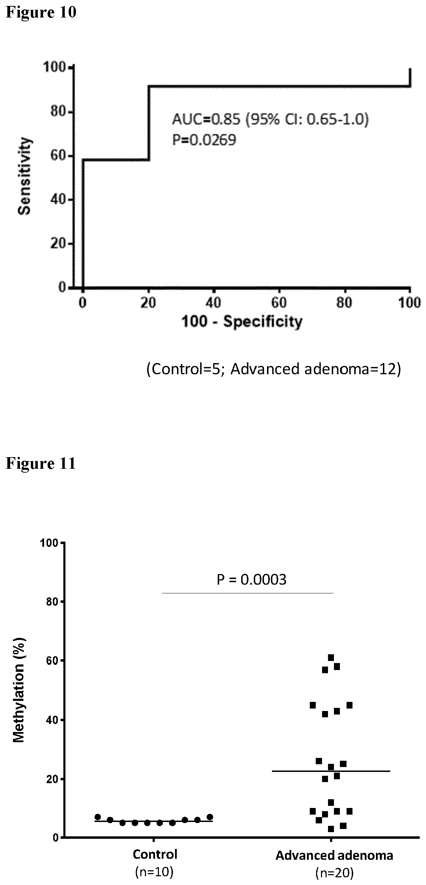

**Figure 10. Receiver Operating Characteristics (ROC) curve for the methylation of *LINC00473* promoter in circulating plasma DNA of advanced colorectal adenomas respect to normal controls.** The area under the ROC curve (AUC) shows the high diagnostic accuracy of the methylation of *LINC00473* promoter to discriminate between advanced colorectal adenomas and healthy controls. P, indicates the p-value of the ROC curve. CI, is the confidence interval.

**Figure 11. DNA methylation levels of *LINC00473* promoter in advanced colorectal adenomas from tissues.** Advanced colorectal adenomas showed significantly higher methylation levels of *LINC00473* than non-tumor controls. The methylation levels of the *LINC00473* promoter were analysed by pyrosequencing and expressed as % of methylation. The horizontal lines represent the median of the methylation levels. P, indicates the p-value analysed by Mann-Whitney U test.

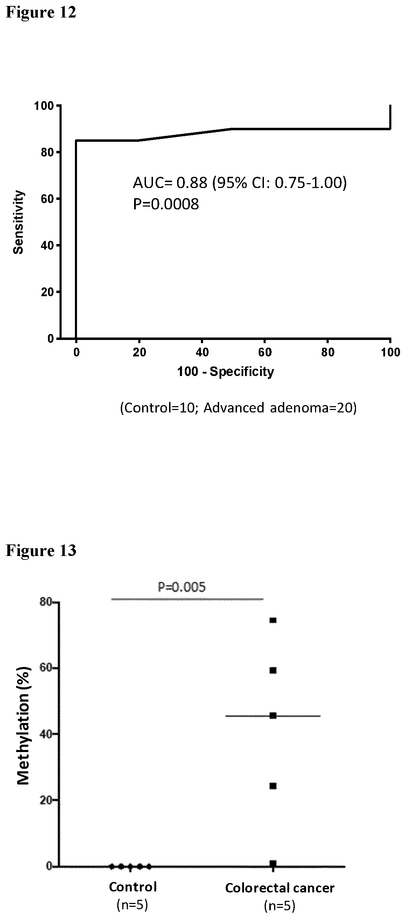

**Figure 12. Receiver Operating Characteristics (ROC) curve for the methylation of *LINC00473* promoter in advanced colorectal adenoma from tissues.** The area under the ROC curve (AUC) shows the high diagnostic accuracy of the methylation of *LINC00473* promoter to discriminate between advanced colorectal adenomas and non-tumor controls. P, indicates the p-value of the ROC curve. CI, is the confidence interval.

**Figure 13. DNA methylation levels of *LINC00473* promoter in circulating plasma DNA of colorectal cancer by droplet digital PCR (ddPCR).** Colorectal cancer patients showed significantly higher methylation levels of *LINC00473* than non-tumor controls. The methylation levels of the *LINC00473* promoter were analysed in triplicates by ddPCR. The horizontal lines represent the median of the methylation levels. P, indicates the p-value analysed by Mann-Whitney U test.

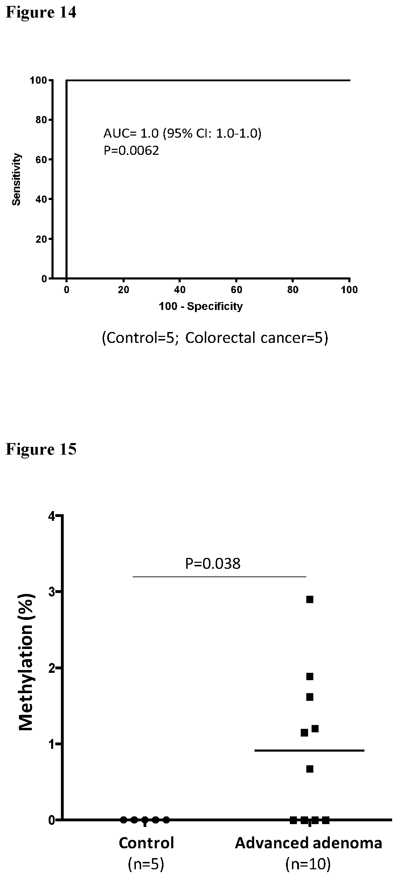

**Figure 14. Receiver Operating Characteristics (ROC) curve for the methylation of *LINC00473* promoter in circulating plasma DNA of colorectal cancer patients respect to normal controls analysed by droplet digital PCR (ddPCR).** The area under the ROC curve (AUC) shows the high diagnostic accuracy of the methylation of *LINC00473* promoter to discriminate between colorectal cancer patients and healthy individuals. P, indicates the p-value of the ROC curve.

**Figure 15. DNA methylation levels of *LINC00473* promoter in circulating plasma DNA of advanced colorectal adenomas by droplet digital PCR.** Advanced colorectal adenoma patients showed significantly higher methylation levels of *LINC00473* than healthy controls. The methylation levels of *LINC00473* were analysed in triplicates by droplet digital PCR. The horizontal lines represent the median of the methylation levels and P indicates the p-value by analysed Mann-Whitney U test.

**Detailed description of the invention**

**Example 1. Methods.**

**Example 1.1. Study participants for colorectal tissue analysis.**

We initially analyzed 3 independent cohorts (Cohort 1, Cohort 2, and Cohort 3) to study the DNA methylation of *LINC00473* in colorectal tissues. Cohort 1 are represented by primary colorectal cancer patients (n=273) and non-tumor controls (n=38). The Cohort 2 includes 180 primary colorectal cancer patients and 93 non-tumor controls. And in the Cohort 3 there are 12 primary colorectal cancer patients, 12 adenomas and 12 non-tumor controls.

Moreover, for the purpose the present PCT application, we also analysed a new independent cohort (Cohort 4) to study the DNA methylation of LINC00473 in colorectal tissues, which includes 50 colorectal adenomas (comprising 20 confirmed advanced colorectal adenomas) and 10 non-tumor controls.

**Example 1.2. Analysis of DNA methylation data of colorectal tissues by 450K array.**

The results of DNA methylation of Cohort 1 were analyzed from the Infinium HumanMethylation450K BeadChip data (450K array) obtained in the public database The Cancer Genome Atlas (TCGA). The 450K array (Illumina) covers > 450,000 CpG sites along the human genome, and methylation score of each CpG is represented as beta ($\beta$)-values that ranged between 0.0 (completely unmethylated) and 1.0 (completely methylated).

**Example 1.3. Bisulfite pyrosequencing analysis of colorectal tissues.**

The DNA methylation status of *LINC00473* promoter in the Cohorts 2, 3 and 4 were analyzed by bisulfite pyrosequencing. After bisulfite conversion of ~500 ng of DNA with EZ-96 DNA Methylation kit (Zymo Research Corp.), the DNA methylation levels of *LINC00473* promoter were analyzed with a PyroMark Q96 System (Qiagen) according to the manufacturer's instructions. CpG site methylation quantification was obtained using Pyro Q-CpG 1.0.9 (Qiagen). Primer sequences (**Table 1**) were designed with PyroMark Assay Design 2.0 (Qiagen). **Table 1** shows the primers for methylation analyses of *LINC00473* by pyrosequencing.

**Table 1**

| Primer sequence (5'-3') | Primer size (nt) |
|---|---|
| F: SEQ ID NO: 1 | 27 |
| R: SEQ ID NO: 2 | 23 |
| S: SEQ ID NO: 3 | 22 |
| *F: Forward; R: Reverse; S: Sequencing; nt: nucleotides* | |

In a preferred embodiment, the primers used in the present invention, preferably the primer of SEQ ID NO: 2, are biotinylated by means of the incorporation of a molecule of biotin.

**Example 1.4. Study participants for liquid biopsy analysis.**

For the analysis of *LINC00473* in liquid biopsy of colorectal cancer, the study included 26 advanced colorectal cancer patients (stage III and IV) prospectively recruited at the time of diagnosis (baseline), before the beginning of the treatment and surgery. Demographic and clinical characteristics of the patients are represented in **Table 2**. Twenty-eight healthy controls were also included in the study. The disease status and staging of colorectal cancer patients was obtained by the medical oncologists of the Oncology Department at Complejo Hospitalario Universitario de Santiago de Compostela (CHUS). **Table 2** shows clinical characteristics of the colorectal cancer patients.

**Table 2**

| Characteristics | Patients (n=26) |
|---|---|
| **Gender** | |
| Male | 17 |
| Female | 9 |
| **Age (years)** | |
| Mean $\pm$ SD | 67 $\pm$ 10 |
| **Stage** | |
| III | 4 |
| IV | 22 |
| **Histology** | |
| Adenocarcinoma | 24 |
| Mucinous carcinoma | 2 |
| SD: Standard deviation | |

For the analysis of *LINC00473* in liquid biopsy of advanced colorectal adenoma, the study included 5 healthy controls and 12 advanced adenoma patients. All the individuals for liquid biopsy analysis were recruited at the Complejo Hospitalario Universitario de Santiago de Compostela (CHUS).

For the analysis of LINC00473 in liquid biopsy of colorectal cancer by droplet digital PCR (ddPCR), the study included 5 advanced colorectal cancer patients prospectively recruited at the time of diagnosis (baseline), before the beginning of the treatment and surgery. Ten advanced adenomas and 5 healthy controls were also included in the study. The patients and controls were obtained at Complejo Hospitalario Universitario de Santiago de Compostela (CHUS).

**Example 1.5. Blood sample collection and plasma isolation.**

Blood samples were obtained by phlebotomy using collection tubes containing EDTA as anticoagulant. Plasma was isolated from blood samples within 2 h of collection with a first centrifugation of blood tubes (1,500g, 10 min, 4°C). Plasma was transferred to a 1.5 mL tubes and a second centrifugation was performed (15,000g, 10 min, 4°C). Plasma was transferred to new 1.5 mL tubes and stored at -80 °C.

**Example 1.6. Isolation of circulating DNA from plasma samples.**

After defrosting the plasma samples, circulating DNA was isolated from 2-3 mL of plasma using the QIAamp® Circulating Nucleic Acid Kit (Qiagen) and the vacuum system QIAvac 24 Plus (Qiagen) following the manufacturer's recommendations. Circulating DNA was finally eluted in 75 $\mu$L of elution buffer and 1 $\mu$L of this elution was used to quantify the concentration of circulating DNA by the kit QuantiFluor® ONE dsDNA with the Quantus™ Fluorometer (Promega). After the quantification, circulating DNA was stored at -80 °C.

**Example 1.7. Bisulfite conversion of circulating DNA.**

For DNA bisulfite conversion, 15-50 ng of circulating DNA were used, and conversion was performed with EZ DNA Methylation-Lightning Kit (Zymo Research) according to manufacturer's recommendations following these thermocycling steps: 98 °C 8 min, 54°C 60 min and 4°C. After column-based purification with the steps of binding, L-desulphonation and washing, bisulfite-converted DNA (bis-DNA) was eluted in 15 $\mu$L of elution buffer and stored at -80 °C.

**Example 1.8. DNA methylation analysis of the *LINC00473* promoter region in liquid biopsy by real-time PCR.**

The methylation status of the promoter region of the long intergenic non-protein coding RNA 473 (*LINC00473; previous symbol C6orfl76*) was analyzed in circulating plasma DNA. The non-coding gene *LINC00473* (NCBI ID: 90632; UCSC ID: uc063sul.1; Ensambl ID: ENSG00000223414; HGNC ID: HGNC:21160) is located in chromosome (chr) 6 at position 6q27, and has a CpG island containing its promoter region, which is located in chr6: 166401527-166402659. In addition, the promoter region *ofLINC00473* overlaps in the genome with the body of the long intergenic non-protein coding RNA 602 or LINC00602 (NCBI ID: 441177; UCSC ID: uc01 1egm.4; Ensambl ID: ENSG00000281832; HGNC ID: HGNC:43917).

The methylation levels of the promoter region of *LINC00473* were determined by real-time PCR with a quantitative methylation-specific PCR assay (qMSP) in a StepOne Plus system (Applied Biosystems). Each reaction contained 2uL of bisulfite-converted DNA (bis-DNA) as a template, 10 μl *Power* SYBR™ Green PCR Master Mix (ThermoFisher) and 150 nM each forward and reverse primers (**Table 3**) to detect methylation or unmethylation in a total volume of 20 μl. Reactions were performed in a *MicroAmp™ Fast Optical 96-Well Reaction Plate* (Applied Biosystems). Thermocycling conditions in StepOne Plus system were: 10 min at 95 °C, followed by 50 cycles of 94 °C for 15 s and 60 °C for 30 s. Water was included as non-template control in each run to verify the absence of contamination during reactions. The colorectal cancer cell line HCT-116 and normal leukocytes (NL) were used in each analysis as positive controls for methylation and unmethylation, respectively. All the samples and controls were analyzed in triplicates. The threshold cycle (Ct) was determined for each reaction with the specific primers for methylation and unmethylation using the software *StepOne Real-Time PCR* (Applied Biosystems). The DNA methylation level of each sample was expressed as a percentage of methylation (%) according to the following calculation based on Cottrell *et al.* (2007):

$$\text{Methylation (\%)} = 100/[1+2^{\wedge}(CT_{CG}-CT_{TG})]$$

In this formula, $CT_{CG}$ represent the threshold cycle of the methylation status and $CT_{TG}$ indicates the threshold cycle of the un-methylation status. With this calculation methylation levels can be between ranges of 0% to 100% of methylation. **Table 3** shows the primers for methylation analyses of *LINC00473* by real-time PCR.

**Table 3**

| Methylation status | Primer sequence (5'-3') | Primer size (nt) | Amplicon size (bp) |
|---|---|---|---|
| Methylated | F: SEQ ID NO: 4 | 20 | 114 [(*)] |
| | R: SEQ ID NO: 5 | 20 | |
| Unmethylated | F: SEQ ID NO: 6 | 23 | 116 [(#)] |
| | R: SEQ ID NO: 7 | 23 | |
| *F: Forward; R: Reverse; nt: nucleotides; bp: base pairs.* <br> *(\*)Position: chr6: 166402364-166402477; [(#)]Position: chr6:166402363-166402478.* | | | |

The forward and reverse primers used for the methylation analysis of *LINC00473* allows to detect the methylation status of 6 CpGs (**Table 4**) located at the promoter region of *LINC00473.* One of the CpGs (chr6: 166402416) contained in the sequence amplified (amplicon) by real-time PCR corresponds with the CpG termed cg08886973 according to the Infinium Human Methylation 450K and Infinium MethylationEPIC assays (Illumina), which are microarray assays able to detect the methylation status of more than 450,000 and 850,000 CpGs, respectively. These microarrays are also able to detect other CpGs from the *LINC00473* promoter identified as cg06545143 (chr6: 166402638) and cg21306006 (chr6: 166402081) that are located, respectively, 162 nucleotides upstream and 282 nucleotides downstream the amplicon of *LINC00473* analyzed in this study. The three CpGs included in this type of microarray assays (cg06545143, cg08886973, cg21306006) are located in the TSS1500 promoter region of *LINC00473,* which is the region of the promoter between 200 and 1500 nucleotides upstream its transcription start site (TSS). All the chromosomal positions previously indicated are according to the version GRCh37/hg19 of the UCSC Genome Browser from the University of California (https://genome.ucsc.edu/index.html). **Table 4** shows the CpGs of *LINC00473* that are detected in the methylation analyses by real-time PCR.

**Table 4**

| Methylation status | Primer | Chromosomal position |
|---|---|---|
| Methylated or Unmethylated | Forward | chr6: 166402474<br>chr6: 166402463<br>chr6: 166402457 |
| | Reverse | chr6: 166402379<br>chr6: 166402375<br>chr6: 166402364 |

**Example 1.9. DNA methylation analysis of the *LINC00473* promoter region in liquid biopsy by droplet digital PCR (ddPCR).**

The methylation levels of the promoter region of*LINC00473* were determined by droplet digital PCR (ddPCR) in a QX200 system (Bio-Rad). First, a multiplex preamplification reaction was performed using ~2 ng of bisulfite-converted DNA (bis-DNA), 25 μl SsoAdvanced™ PreAmp Supermix (Bio-Rad), 0.5 μl of a custom Bio-Rad assay containing the forward and reverse primers and the probe for methylation (Bio-Rad) (**Table 5**), and 0.5 μl of a custom Bio-Rad assay containing the forward and reverse primers and the probe for unmethylation (**Table 5**), in a total volume of 50 μl. Final volume was completed with water. Reactions were performed in 0.2 ml PCR tubes (Axygen) on a ProFlex PCR System (Applied Biosystems): 3 min at 95 °C, 10 cycles of 95 °C for 15 s and 56.2 °C for 4 min; and a final hold step of 4 °C.

Next, a multiplex mix reaction was performed containing 2 uL of the pre-amplification product previously obtained, 11 μl ddPCR Supermix for Probes (No dUTP) (Bio-Rad), 2.2 μl of a custom Bio-Rad assay containing the forward and reverse primers and the probe for methylation (**Table 5**), and 2.2 μl of a custom Bio-Rad assay containing the forward and reverse primers and the probe for unmethylation (Bio-Rad) (**Table 5**), in a total volume of 22 μl. Final volume was completed with water. Then, 20 uL of each mix reaction and 70 μl of Droplet Generation oil (Bio-Rad) were transferred, respectively, to the sample and oil wells of a DG8™ Cartridge (BIO-RAD) and loaded into the QX200™ Droplet Generator (Bio-Rad). Drops were generated into the droplet well of the cartridge, transferred to a ddPCR 96-well plate (Bio-Rad) and loaded in a C1000 Touch Thermal Cycler (Bio-Rad): 10 min at 95 °C, 40 cycles of 95 °C for 15 s and 56.2 °C for 30 s; 98 °C for 10 min and a final hold step of 4 °C. The temperature ramp increment was 2.5 °C/s for all steps. Then, the 96-well plate was read on a QX200™ Droplet Reader (Bio-Rad). Water was included as non-template control in each run to verify the absence of contamination during reactions. The colorectal cancer cell line HCT-116 and normal leukocytes (NL) were used in each analysis as positive controls for methylation and unmethylation, respectively. All controls were included in each plate with and without the preamplification step. All the samples and controls were analysed in triplicates. Analysis of the data was performed with the QuantaSoft software (Bio-Rad).

The DNA methylation level of each sample was expressed as a percentage of methylation (%) according to the following calculation:

$$\text{Methylation (\%)} = [M / (U + M)] \times 100$$

In this formula, M (Methylation) represents the copies/μl of the target DNA molecule for the methylation probe (FAM probe), and U (Unmethylation) indicates the copies/μl of the target DNA molecule for the unmethylation probe (HEX probe). With this calculation methylation levels can be between ranges of 0% to 100% of methylation. **Table 5** shows the sequences of primers and probes for methylation analyses of *LINC00473* by ddPCR. Based on these sequences two custom Bio-Rad assays containing the primers and probe for methylation and unmethylation, respectively, were obtained from Bio-Rad according to manufacturer's recommendations: Primer to probe ratio: 1.8, primer concentration in final reaction 450 nM and probe concentration in final reaction 250 nM.

**Table 5**

| Methylation status | Primer and probe sequence (5'-3') | Primer/probe size (nt) | Amplicon size (bp) |
|---|---|---|---|
| Methylated | F: SEQ ID NO: 4 | 20 | 114 [*] |
| | R: SEQ ID NO: 5 | 20 | |
| | P: FAM-SEQ ID NO: 8 | 20 | |

(continued)

| Methylation status | Primer and probe sequence (5'-3') | Primer/probe size (nt) | Amplicon size (bp) |
|---|---|---|---|
| Unmethylated | F: SEQ ID NO: 6 | 23 | 116[#] |
| | R: SEQ ID NO: 7 | 23 | |
| | P: HEX- SEQ ID NO: 9 | 27 | |

*F: Forward; R: Reverse; P: Probe; nt: nucleotides; bp: base pairs. FAM: fluorescein; HEX: hexachloro-fluoresceine* [*] *Position: chr6: 166402364-166402477;* [#] *Position: chr6:166402363-166402478.*

The forward and reverse primers, and the probe used for the methylation analysis of LINC00473 by ddPCR allows to detect the methylation status of 9 CpGs (**Table 6**) located at the promoter region of *LINC00473*. One of the CpGs (chr6: 166402416), which corresponds with the CpG termed cg08886973 according to the Infinium Human Methylation 450K and Infinium MethylationEPIC assays (Illumina), is contained in the sequence amplified (amplicon) and is detected with the methylated and unmethylated probe indicated in **Table 5**. All the CpGs positions of *LINC00473* that are detected in the methylation analyses by ddPCR are represented in **Table 6**. All the chromosomal positions indicated in **Table 6** are according to the version GRCh37/hg19 of the UCSC Genome Browser from the University of California (https://genome.ucsc.edu/index.html).

**Table 6**

| Methylation status | Primer | Chromosomal position |
|---|---|---|
| Methylated or Unmethylated | Forward | chr6: 166402474<br>chr6: 166402463<br>chr6: 166402457 |
| | Probe | chr6: 166402416<br>chr6: 166402411<br>chr6: 166402400 |
| | Reverse | chr6: 166402379<br>chr6: 166402375<br>chr6: 166402364 |

**Example 1.10. Statistical analyses.**

The Kolmogorov-Smirnov test was first used to evaluate the normality of the distribution of the data. Subsequently, the nonparametric Mann-Whitney U test was used for the comparison of the data. To assess the diagnostic accuracy of *LINC00473* methylation to detect colorectal cancer a Receiver Operating Characteristic (ROC) curve was performed. The Youden index (J), which allows to obtain the cutoff point for methylation that provides the greatest combination of sensitivity and specificity, was calculated according to the formula: J = Sensitivity + Specificity-1 (Fluss et al., 2005). To measure the effectiveness of the diagnostic test, the positive (PPV) and negative predictive value (NPV) were calculated: PPV = true positive / (true positive + false positive); NPV = true negative / (true negative + false negative) (Hajian-Tilaki, 2013). To evaluate the survival analysis the Kaplan-Meier method was performed and the log-rank (Mantel-Cox) test was used to examine differences between the methylated and unmethylated group. The SPSS or GraphPad Prism 7.0 software was used for statistical analysis and graphic representation. All expressed p-values were calculated with two-tailed tests and were considered significant when $p < 0.05$.

**Example 2. Results.**

**Example 2.1. Tissue samples.**

**Example 2.1.1. The methylation analysis of the promoter of *LINC00473* in tumor tissues allows detecting colorectal cancer patients.**

The DNA methylation levels of *LINC00473* promoter region of colorectal tissue samples (273 colorectal cancer patients at stage I, II, III and IV; and 38 non-tumor controls) were obtained after the analysis by Infinium HumanMethylation450K BeadChip (450K array) from the international public database The Cancer Genome Atlas (TCGA) (Cohort 1). In this analysis the methylation data of 450K array are expressed as average beta ($\beta$)-values, which are defined from 0.0 to 1.0. We focused our analysis in the CpG of *LINC00473* promoter region identified in the 450K array as cg08886973. This analysis showed significantly (p<0.0001) higher methylation levels in colorectal cancer patients (0.51 $\pm$ 0.011) than in non-tumor controls (0.11 $\pm$ 0.006) (**Figure 1A**). Similar results were obtained when we analyzed the methylation status of the *LINC00473* promoter in the Cohort 2 by bisulfite pyro sequencing (180 colorectal cancer patients: 33.93% $\pm$ 1.51%; 93 non-tumor controls: 5.84% $\pm$ 0.78%; p<0,0001) (**Figure 1B**). In this case, the methylation values obtained from pyrosequencing were expressed in percentage of methylation (%), which is defined from 0% to 100%. Altogether these data indicate that the methylation levels of the promoter region of *LINC00473* analyzed in colorectal tissues are able to differentiate between non-tumor individuals and patients with colorectal cancer.

The analysis of the methylation levels of *LINC00473* in the colorectal tissue samples from the Cohort 1 by receiver operating characteristic curve (ROC) showed a very high diagnostic accuracy to differentiate colorectal cancer patients (I, II, III and IV) from non-tumor controls with an area under the curve (AUC) of 0.94 (p<0.0001; CI 95%: 0.91-0.97) (**Figure 2A**). Similar results were obtained from the Cohort 2 (AUC=0.90; p<0.0001; CI 95%: 0.86-0.94) (**Figure 2B**). These results indicate that the analysis of the methylation status of the *LINC00473* promoter region in colorectal tissue samples has a great diagnostic accuracy to identify colorectal cancer patients.

Importantly, when we stratified the colorectal cancer patients according to their stage in the Cohort 1 (I: n=41; II: n=106; III: n=58; IV: n=35) and Cohort 2 (I: n=10; II: n=84; III: n=56; IV: n=28) (**Figure 3**), the results also showed significantly (p<0.0001) higher methylation levels in each stage of colorectal cancer patients compared to non-tumor controls (Cohort 1, n=38; Cohort 2, n= 93).

**Example 2.1.2. The methylation analysis of the promoter of *LINC00473* in colorectal tissues allows the identification of adenomas and advanced colorectal adenomas.**

The DNA methylation levels of *LINC00473* promoter region in colorectal tissues of 12 non-tumor controls, 12 adenomas and 12 colorectal cancer patients (Cohort 3) were analyzed by bisulfite pyro sequencing and expressed as % of methylation. These results showed significantly (p=0.0036) higher methylation levels in adenomas (28.21% $\pm$ %) than in healthy controls (7.28% $\pm$ 0.81%) (**Figure 4**). As expected, the colorectal cancer patients (32.83% $\pm$ 5.19%) also showed significantly (P=0.002) differences in methylation levels respect to the non-tumor controls. Importantly, the methylation levels of *LINC00473* in adenomas were similar to colorectal cancer patients (P=0.3186). These data indicate that the methylation levels of the promoter region of *LINC00473* analyzed in colorectal tissues are able to differentiate between non-tumor individuals and patients with adenomas, suggesting that the methylation status of the *LINC00473* promoter can be useful for the early detection of colorectal cancer.

The analysis of the methylation levels of *LINC00473* in the colorectal tissue samples from non-tumor and adenoma samples of Cohort 3 by receiver operating characteristic curve (ROC) showed a very high diagnostic accuracy to differentiate adenomas from non-tumor controls with an area under the curve (AUC) of 0.84 (p=0,0047; 95% CI: 0.66-1,00) (**Figure 5**). These results indicate that the analysis of the methylation status of the *LINC00473* promoter region in colorectal tissue samples has a great diagnostic accuracy to identify patients with colorectal adenomas, which are at risk of developing colorectal cancer.

Moreover, in order to evaluate the methylation status of colorectal adenomas and advanced colorectal adenomas, we analysed the DNA methylation levels of *LINC00473* promoter region by bisulfite pyrosequencing in a new independent cohort of tissue samples with 10 non-tumor controls and 50 adenomas, which included 20 confirmed advanced colorectal adenomas. This analysis showed significantly (p<0.0001) higher methylation levels in adenomas (20.96% $\pm$ 15.33%) than in healthy controls (5.70% $\pm$ 0.82%). Specifically, this analysis showed significantly (p<0.0003) higher methylation levels in advanced colorectal adenomas (26.35% $\pm$ 19.66%) than in controls (5.70% $\pm$ 0.82%) (**Figure 11**). In fact, the receiver operating characteristic curve (ROC) analysis also showed a very high diagnostic accuracy to differentiate advanced colorectal adenomas from non-tumor controls with an AUC of 0.88 (p< 0.0008; 95% CI: 0.75-1.00) (**Figure 12**). These results confirm that the methylation levels of the promoter region of *LINC00473* are useful for the detection of adenomas, specifically for the detection of advanced colorectal adenomas.

**Example 2.2. Liquid biopsy**

**Example 2.2.1. The methylation analysis of the promoter of *LINC00473* in liquid biopsy detects colorectal cancer patients.**

The DNA methylation levels (%) of *LINC00473* promoter region in plasma of 28 controls and 26 colorectal cancer patients were analyzed by real-time PCR showing significantly (p<0.0001) higher methylation levels in colorectal cancer patients (25.87% $\pm$ 32.73%) than in healthy controls (0.003% $\pm$ 0.009%) (**Figure 6**). Higher methylation levels of *LINC00473* were detected in 21 out of 26 (81%) colorectal cancer patients respect to healthy individuals. These data indicate that the methylation levels of the promoter region *ofLINC00473* analyzed in liquid biopsy are able to differentiate between healthy individuals and patients with colorectal cancer.

The analysis of the methylation levels of *LINC00473* in plasma by receiver operating characteristic curve (ROC) showed a very high diagnostic accuracy to differentiate colorectal cancer patients from healthy controls with an area under the curve (AUC) of 0.87 (p<0.0001; CI 95%: 0.76-0.99) (**Figure 7**). Based on the ROC curve analysis and using the value of Youden's index, a methylation cutoff for *LINC00473* in plasma of 0.30% was obtained, allowing the detection of colorectal cancer with a sensitivity of 81% (CI 95%: 61%-93%) and a specificity of 100% (CI 95%: 88-100) (**Table 7**). Using this methylation level cutoff, a positive predictive value (PPV) of 100% and a negative predictive value (NPV) of 85% was obtained for the detection of colorectal cancer. These results indicate that the non-invasive analysis in liquid biopsy of the methylation status of the *LINC00473* promoter region has a great diagnostic accuracy to identify colorectal cancer patients. **Table 7** shows the characteristics of the methylation analysis of *LINC00473* promoter region as a diagnostic test for colorectal cancer detection.

**Table 7**

| Parameters | Percentage (%) |
|---|---|
| Sensitivity | 81 |
| Specificity | 100 |
| VPP | 100 |
| VPN | 85 |
| VPP: Positive predictive value; VPN: Negative predictive value | |

Moreover, DNA methylation levels (%) of *LINC00473* promoter region in plasma of colorectal cancer patients were also evaluated using the droplet digital PCR (ddPCR) methodology. For this purpose, we used 5 controls and 5 colorectal cancer patients. These analysis showed significantly (p=0.005) higher methylation levels in colorectal cancer patients (41.19% $\pm$ 28.79%) than in healthy controls (0.0% $\pm$ 0.0%) (**Figure 13**). Higher methylation levels of *LINC00473* were detected in 5 out of 5 (100%) colorectal cancer patients respect to healthy individuals, indicating that the methylation levels of the promoter region of *LINC00473* analyzed in liquid biopsy by ddPCR are also able to differentiate between healthy individuals and patients with colorectal cancer. In addition, the receiver operating characteristic curve (ROC) analysis showed that the methylation levels *ofLINC00473* in plasma obtained by ddPCR had a very high diagnostic accuracy to differentiate colorectal cancer patients from healthy controls with an area under the curve (AUC) of 1.0 (p<0.0062; CI 95%: 1.0-1.0) (**Figure 14**). These results indicate that the non-invasive analysis in liquid biopsy of the methylation status of the *LINC00473* promoter region by ddPCR has a great diagnostic accuracy to identify colorectal cancer patients.

**Example 2.2.2. DNA methylation levels of the promoter region of *LINC00473* in liquid biopsy predict the outcome of colorectal cancer patients.**

The clinical impact on survival of the methylation levels of *LINC00473* promoter was studied in plasma samples of 25 colorectal cancer patients from **Table 2** with available clinical data. Colorectal cancer patients were classified respect to the median methylation level of the group (methylation level of 10%) as presenting i) High level of methylation in *LINC00473* (H: methylation level >10%) or ii) Low level of methylation in *LINC00473* (L: methylation level <10%). According to this classification of the patients, Kaplan-Meir analysis showed that the presence of high levels of methylation in *LINC00473* was significantly associated with shorter overall survival (OS) [hazard ratio (HR) =3.37, 95% confidence interval (CI) =1.19-9.93, P = 0.0256] (**Figure 8**). These results show that the methylation status of *LINC00473* in liquid biopsy is able to predict the clinical outcome of colorectal cancer patients, suggesting that the analysis of *LINC00473* methylation in plasma could be useful as a prognostic biomarker at the time of colorectal cancer diagnosis.

**Example 2.2.3. The methylation analysis of the promoter of *LINC00473* in liquid biopsy detects advanced colorectal adenoma patients.**

The DNA methylation levels (%) of *LINC00473* promoter region in plasma of 5 healthy controls and 12 advanced colorectal adenoma patients were analyzed by real-time PCR showing significantly (p=0.0262) higher methylation levels in advanced colorectal adenoma patients (0.0676% $\pm$ 0.1195%) than in healthy controls (0.0001% $\pm$ 0.0002%) (**Figure 9**). These data indicate that the methylation levels of the promoter region of *LINC00473* analyzed in liquid biopsy are able to differentiate between healthy individuals and patients with advanced colorectal adenoma.

The analysis of the methylation levels of *LINC00473* in plasma by receiver operating characteristic curve (ROC) showed a very high diagnostic accuracy to differentiate patients with advanced colorectal adenoma from healthy controls with an area under the curve (AUC) of 0.85 (p=0.00269; CI 95%: 0.65-1.0) (**Figure 10**). Based on the ROC curve analysis, a methylation cutoff for *LINC00473* in plasma of 0.000115 was obtained, allowing the detection of advanced adenoma with a sensitivity of 92% and a specificity of 80% (**Table 8**). Using this methylation level cutoff, a PPV of 92% and a NPV of 80% was obtained for the detection of advanced colorectal adenoma. These results indicate that the non-invasive analysis in liquid biopsy of the methylation status of the *LINC00473* promoter region has a great diagnostic accuracy to identify patients with advanced colorectal adenomas. **Table 8** shows the characteristics of the methylation analysis of *LINC00473* promoter region as a diagnostic test for the detection of advanced colorectal adenoma.

**Table 8**

| Parameters | Percentage (%) |
|---|---|
| Sensitivity | 92 |
| Specificity | 80 |
| VPP | 92 |
| VPN | 80 |
| VPP: Positive predictive value; VPN: Negative predictive value | |

Moreover, DNA methylation levels (%) of *LINC00473* promoter region in plasma were also evaluated by droplet digital PCR in 5 healthy controls and 10 advanced colorectal adenoma patients. These results showed significantly (p=0.038) higher methylation levels in advanced colorectal adenoma patients (0.94% $\pm$ 0.99%) than in healthy controls (0.0% $\pm$ 0.0%) (**Figure 15**), indicating that the methylation levels of the promoter region of *LINC00473* analysed in liquid biopsy by ddPCR are also able to differentiate between healthy individuals and patients with advanced colorectal adenoma.

**Claims**

1. *In vitro* method for the diagnosis of colorectal cancer and/or a pre-cancerous stage thereof, which comprises determining the methylation status of at least the gene *LINC00473* in a liquid biopsy obtained from the patient, wherein a higher level of methylation of the gene *LINC00473*, as compared with a reference level of methylation of the gene *LINC00473* measured in healthy control subjects, is an indication that the subject is suffering from colorectal cancer and/or a pre-cancerous stage thereof.

2. *In vitro* method for the prognosis of colorectal cancer, or for predicting the clinical outcome of colorectal cancer patients, which comprises determining the methylation status of at least the gene *LINC00473 in* a liquid biopsy obtained from the patient, wherein a higher level of methylation of the gene *LINC00473*, as compared with a reference level of methylation of the gene *LINC00473* measured in control patients, is an indication that the patient has a poor prognosis or poor clinical outcome.

3. *In vitro* method, according to any of the previous claims, wherein the liquid biopsy is a sample of plasma, blood or serum.

4. *In vitro* method, according to any of the previous claims, wherein the pre-cancerous stage is colorectal adenoma, preferably advanced colorectal adenoma.

5. *In vitro* method, according to any of the previous claims, wherein the methylation status of the gene *LINC00473* is determined in at least a CpG of the promoter region.

6. *In vitro* method, according to any of the previous claims, wherein the methylation status of the gene *LINC00473* is determined in at least a CpG of the promoter region located between the chromosomal positions Chr6: 166402081 and Chr6: 166402638.

7. *In vitro* method, according to any of the previous claims, wherein the methylation status of the gene *LINC00473* is determined in at least a CpG of the promoter region located at a chromosomal position selected from the group comprising: Chr6: 166402638, and/or Chr6: 166402474, and/or Chr6: 166402463, and/or Chr6: 166402457, and/or Chr6: 166402416, and/or Chr6: 166402379, and/or Chr6: 166402375, and/or Chr6: 166402364, and/or Chr6: 166402081, preferably a CpG selected from the group comprising: cg06545143, and/or cg08886973 and/or cg21306006.

8. *In vitro* method, according to any of the previous claims, wherein the diagnosis of the colorectal cancer and/or a pre-cancerous stage thereof is confirmed by an image technique, preferably colonoscopy.

9. *In vitro* use of at least the methylation status of the gene *LINC00473* for the diagnosis and/or prognosis and/or for predicting the clinical outcome of colorectal cancer and/or a pre-cancerous stage thereof.

10. *In vitro* use of a kit comprising reagents for the determination of at least the methylation status of the gene *LINC00473* for the diagnosis and/or prognosis and/or for predicting the clinical outcome of colorectal cancer and/or a pre-cancerous stage thereof.

11. *In vitro* use, according to any of the claims 9 or 10, wherein the pre-cancerous stage is colorectal adenoma, preferably advanced colorectal adenoma.

12. *In vitro* use, according to any of the claims 9 to 11, wherein the methylation status of the gene *LINC00473* is determined in at least a CpG of the promoter region.

13. *In vitro* use, according to any of the claims 9 to 12, wherein the methylation status of the gene *LINC00473* is determined in at least a CpG of the promoter region located between the chromosomal positions Chr6: 166402081 and Chr6: 166402638.

14. *In vitro* use, according to any of the claims 9 to 13, wherein the methylation status of the gene *LINC00473* is determined in at least a CpG of the promoter region located at a chromosomal position selected from the group comprising: Chr6: 166402638, and/or Chr6: 166402474, and/or Chr6: 166402463, and/or Chr6: 166402457, and/or Chr6: 166402416, and/or Chr6: 166402379, and/or Chr6: 166402375, and/or Chr6: 166402364, and/or Chr6: 166402081, preferably a CpG selected from the group comprising: cg06545143, and/or cg08886973 and/or cg21306006.

15. *In vitro* use, according to any of the claims 9 to 14, wherein the diagnosis of the colorectal cancer and/or a pre-cancerous stage thereof is confirmed by an image technique, preferably colonoscopy.

**Patentansprüche**

1. *In-vitro*-Verfahren zur Diagnose von kolorektalem Krebs und/oder einem präkanzerösen Stadium davon, welches das Bestimmen des Methylierungsstatus mindestens des Gens *LINC00473* in einer Flüssigbiopsie erhalten aus dem Patienten umfasst, wobei ein höheres Methylierungsniveau des Gens *LINC00473,* im Vergleich zu einem Referenz-Methylierungsniveau des Gens *LINC00473,* welches in gesunden Kontrollindividuen gemessen wird, ein Anzeichen dafür ist, dass das Individuum an kolorektalem Krebs und/oder einem präkanzerösen Stadium davon leidet.

2. *In-vitro*-Verfahren zur Prognose von kolorektalem Krebs oder zur Vorhersage des klinischen Ergebnisses von Patienten mit kolorektalem Krebs, welches das Bestimmen des Methylierungsstatus mindestens des Gens *LINC00473* in einer Flüssigbiopsie erhalten aus dem Patienten umfasst, wobei ein höheres Methylierungsniveau des Gens *LINC00473,* im Vergleich zu einem Referenz-Methylierungsniveau des Gens *LINC00473,* welches in Kontrollindividuen gemessen wird, ein Anzeichen dafür ist, dass der Patient eine schlechte Prognose oder ein schlechtes klinisches Ergebnis hat.

3. *In-vitro*-Verfahren nach einem der vorhergehenden Ansprüche, wobei die Flüssigbiopsie eine Probe von Plasma, Blut oder Serum ist.

4. *In-vitro*-Verfahren nach einem der vorhergehenden Ansprüche, wobei das präkanzeröse Stadium kolorektales Adenom, vorzugsweise fortgeschrittenes kolorektales Adenom, ist.

5. *In-vitro*-Verfahren nach einem der vorhergehenden Ansprüche, wobei der Methylierungsstatus des Gens *LINC00473* in mindestens einem CpG der Promoterregion bestimmt wird.

6. *In-vitro*-Verfahren nach einem der vorhergehenden Ansprüche, wobei der Methylierungsstatus des Gens *LINC00473* in mindestens einem CpG der Promoterregion, welche sich zwischen den Chromosomenpositionen Chr6: 166402081 und Chr6: 166402638 befindet, bestimmt wird.

7. *In-vitro*-Verfahren nach einem der vorhergehenden Ansprüche, wobei der Methylierungsstatus des Gens *LINC00473* in mindestens einem CpG der Promoterregion, welche sich in einer Chromosomenposition befindet, ausgewählt aus der Gruppe umfassend: Chr6: 166402638 und/oder Chr6: 166402474 und/oder Chr6: 166402463 und/oder Chr6: 166402457 und/oder Chr6: 166402416 und/oder Chr6: 166402379 und/oder Chr6: 166402375 und/oder Chr6: 166402364 und/oder Chr6: 166402081, vorzugsweise einem CpG ausgewählt aus der Gruppe umfassend: cg06545143 und/oder cg08886973 und/oder cg21306006, bestimmt wird.

8. *In-vitro*-Verfahren nach einem der vorhergehenden Ansprüche, wobei die Diagnose des kolorektalen Krebses und/oder eines präkanzerösen Stadiums davon mittels einer Bildtechnik, vorzugsweise Koloskopie, bestätigt wird.

9. *In-vitro*-Verwendung von mindestens des Methylierungsstatus des Gens *LINC00473* zur Diagnose und/oder Prognose und/oder zur Vorhersage des klinischen Ergebnisses von kolorektalem Krebs und/oder einem präkanzerösen Stadium davon.

10. *In-vitro*-Verwendung eines Kits umfassend Reagenzien zur Bestimmung mindestens des Methylierungsstatus des Gens *LINC00473* zur Diagnose und/oder Prognose und/oder zur Vorhersage des klinischen Ergebnisses von kolorektalem Krebs und/oder einem präkanzerösen Stadium davon.

11. *In-vitro*-Verwendung nach einem der Ansprüche 9 oder 10, wobei das präkanzeröse Stadium kolorektales Adenom, vorzugsweise fortgeschrittenes kolorektales Adenom, ist.

12. *In-vitro*-Verwendung nach einem der Ansprüche 9 bis 11, wobei der Methylierungsstatus des Gens *LINC00473* in mindestens einem CpG der Promoterregion bestimmt wird.

13. *In-vitro*-Verwendung nach einem der Ansprüche 9 bis 12, wobei der Methylierungsstatus des Gens *LINC00473* in mindestens einem CpG der Promoterregion, welche sich zwischen den Chromosomenpositionen Chr6: 166402081 und Chr6: 166402638 befindet, bestimmt wird.

14. *In-vitro*-Verwendung nach einem der Ansprüche 9 bis 13, wobei der Methylierungsstatus des Gens *LINC00473* in mindestens einem CpG der Promoterregion, welche sich in einer Chromosomenposition befindet, ausgewählt aus der Gruppe umfassend: Chr6: 166402638 und/oder Chr6: 166402474 und/oder Chr6: 166402463 und/oder Chr6: 166402457 und/oder Chr6: 166402416 und/oder Chr6: 166402379 und/oder Chr6: 166402375 und/oder Chr6: 166402364 und/oder Chr6: 166402081, vorzugsweise einem CpG ausgewählt aus der Gruppe umfassend: cg06545143 und/oder cg08886973 und/oder cg21306006, bestimmt wird.

15. *In-vitro*-Verwendung nach einem der Ansprüche 9 bis 14, wobei die Diagnose des kolorektalen Krebses und/oder eines präkanzerösen Stadiums davon mittels einer Bildtechnik, vorzugsweise Koloskopie, bestätigt wird.

**Revendications**

1. Procédé *in vitro* pour le diagnostic du cancer colorectal et/ou d'un stade précancéreux associé, qui comprend déterminer le statut de méthylation au moins du gène *LINC00473* dans une biopsie liquide obtenue du patient, dans lequel un niveau de méthylation plus élevé du gène *LINC00473,* par rapport à un niveau de méthylation de référence du gène *LINC00473* mesuré chez des sujets témoins sains, est une indication que le sujet souffre d'un cancer colorectal et/ou d'un stade précancéreux associé.

2. Procédé *in vitro* pour le pronostic du cancer colorectal, ou pour prédire le résultat clinique des patients atteints de cancer colorectal, qui comprend déterminer le statut de méthylation au moins du gène *LINC00473* dans une biopsie liquide obtenue du patient, dans lequel un niveau de méthylation plus élevé du gène *LINC00473,* par rapport à un niveau de méthylation de référence du gène *LINC00473* mesuré chez des patients témoins, est une indication que le patient a un pronostic défavorable ou un résultat clinique défavorable.

3. Procédé *in vitro,* selon l'une quelconque des revendications précédentes, dans lequel la biopsie liquide est un échantillon de plasma, de sang ou de sérum.

4. Procédé *in vitro,* selon l'une quelconque des revendications précédentes, dans lequel le stade précancéreux est un adénome colorectal, de préférence un adénome colorectal avancé.

5. Procédé *in vitro,* selon l'une quelconque des revendications précédentes, dans lequel le statut de méthylation du gène *LINC00473* est déterminé dans au moins un CpG de la région promotrice.

6. Procédé *in vitro,* selon l'une quelconque des revendications précédentes, dans lequel le statut de méthylation du gène *LINC00473* est déterminé dans au moins un CpG de la région promotrice située entre les positions chromosomiques Chr6 : 166402081 et Chr6 : 166402638.

7. Procédé *in vitro,* selon l'une quelconque des revendications précédentes, dans lequel le statut de méthylation du gène *LINC00473* est déterminé dans au moins un CpG de la région promotrice située dans une position chromosomique sélectionnée parmi le groupe comprenant : Chr6 : 166402638, et/ou Chr6 : 166402474, et/ou Chr6 : 166402463, et/ou Chr6 : 166402457, et/ou Chr6 : 166402416, et/ou Chr6 : 166402379, et/ou Chr6 : 166402375, et/ou Chr6 : 166402364, et/ou Chr6 : 166402081, de préférence un CpG sélectionné parmi le groupe comprenant : cg06545143, et/ou cg08886973 et/ou cg21306006.

8. Procédé *in vitro* selon l'une quelconque des revendications précédentes, dans lequel le diagnostic du cancer colorectal et/ou d'un stade précancéreux associé est confirmé par une technique d'imagerie, de préférence colonoscopie.

9. Utilisation *in vitro* au moins du statut de méthylation du gène *LINC00473* pour le diagnostic et/ou le pronostic et/ou pour prédire le résultat clinique du cancer colorectal et/ou d'un stade précancéreux associé.

10. Utilisation *in vitro* d'un kit comprenant des réactifs pour la détermination au moins du statut de méthylation du gène *LINC00473* pour le diagnostic et/ou le pronostic et/ou pour prédire le résultat clinique d'un cancer colorectal et/ou d'un stade précancéreux associé.

11. Utilisation *in vitro,* selon l'une quelconque des revendications 9 ou 10, dans lequel le stade précancéreux est un adénome colorectal, de préférence un adénome colorectal avancé.

12. Utilisation *in vitro,* selon l'une quelconque des revendications 9 à 11, dans lequel le statut de méthylation du gène *LINC00473* est déterminé dans au moins un CpG de la région promotrice.

13. Utilisation *in vitro,* selon l'une quelconque des revendications 9 à 12, dans lequel le statut de méthylation du gène *LINC00473* est déterminé dans au moins un CpG de la région promotrice située entre les positions chromosomiques Chr6 : 166402081 et Chr6 : 166402638.

14. Utilisation *in vitro,* selon l'une quelconque des revendications 9 à 13, dans lequel le statut de méthylation du gène *LINC00473* est déterminé dans au moins un CpG de la région promotrice située dans une position chromosomique sélectionnée parmi le groupe comprenant : Chr6 : 166402638, et/ou Chr6 : 166402474, et/ou Chr6 : 166402463, et/ou Chr6 : 166402457, et/ou Chr6 : 166402416, et/ou Chr6 : 166402379, et/ou Chr6 : 166402375, et/ou Chr6 : 166402364, et/ou Chr6 : 166402081, de préférence un CpG sélectionné parmi le groupe comprenant : cg06545143, et/ou cg08886973 et/ou cg21306006.

15. Utilisation *in vitro,* selon l'une quelconque des revendications 9 à 14, dans lequel le diagnostic du cancer colorectal et/ou d'un stade précancéreux associé est confirmé par une technique d'imagerie, de préférence colonoscopie.

**Figure 1**

**A)**

**B)**

**Figure 2**

A)

(Control=38; Colorectal cancer=273)

B)

(Control=93; Colorectal cancer=180)

**Figure 3**

**A)**

**B)**

**Figure 4**

**Figure 5**

(Control=12; Adenoma=12)

Figure 6

Figure 7

(Control=28; Colorectal cancer=26)

Figure 8

Figure 9

Figure 10

(Control=5; Advanced adenoma=12)

Figure 11

Figure 12

AUC= 0.88 (95% CI: 0.75-1.00)
P=0.0008

(Control=10; Advanced adenoma=20)

Figure 13

**Figure 14**

AUC= 1.0 (95% CI: 1.0-1.0)
P=0.0062

(Control=5; Colorectal cancer=5)

**Figure 15**

P=0.038

Control
(n=5)

Advanced adenoma
(n=10)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LI et al.** *Oncogene,* 2014
- **CHEN et al.** *Biochemical and Biophysical Research communications,* 2018
- **REX DK ; BOLAND CR ; DOMINITZ JA ; GIARDIELLO FM ; JOHNSON DA ; KALTENBACH T ; LEVIN TR ; LIEBERMAN D ; ROBERTSON DJ.** Colorectal Cancer Screening: Recommendations for Physicians and Patients From the U.S. Multi-Society Task Force on Colorectal Cancer. *Gastroenterology,* 09 June 2017, vol. 153 (1), 307-323
- **EAST JE ; ATKIN WS ; BATEMAN AC ; CLARK SK ; DOLWANI S ; KET SN ; LEEDHAM SJ ; PHULL PS ; RUTTER MD ; SHEPHERD NA.** British Society of Gastroenterology position statement on serrated polyps in the colon and rectum. *Gut.,* 27 April 2017, vol. 66 (7), 1181-1196